# EUROPEAN PATENT APPLICATION

(11) **EP 2 457 572 A1**
(43) Date of publication of application: **30.05.2012**
(21) Application number: 10382289.6
(22) Date of filing: 05.11.2010
(51) Int. Cl.: A61K 31/4745, A61K 33/24, A61K 33/26, A61K 33/34, A61K 33/38, A61K 45/06, A61P 35/00, A61P 35/04, A61P 31/12, A61P 31/16, A61P 31/18, A61P 31/22, A61P 37/00, A61P 37/06, A61P 19/02, B22F 1/00

(54) **Use of atomic quantum clusters (aqcs) in the prevention of cell proliferative disorders, viral infections and autoimmune diseases**

(71) Applicant: Universidade De Santiago De Compostela, 15782 Santiago de Compostela (ES); Nanogap Sub-Nmnm-Powders, 15895 Ames (ES)
(72) Inventor: López Quintela, Manuel Arturo, 15782, Santiago de Compostela (ES); Domínguez Puente, Fernando, 15782, Santiago de Compostela (ES)
(74) Representative: ABG Patentes, S.L.

(57) **Abstract**

The present invention relates to stable atomic quantum cluster (AQC), a pharmaceutically acceptable salt, a derivative or a hydrate thereof, characterized in that said AQC, pharmaceutically acceptable salt, derivative or hydrate thereof has a thickness of less than 0.50 nm, for use as chemotherapy agent in the prevention and/or treatment of viral infections, autoimmune diseases and cell proliferative disorders, with the exclusion of breast cancer, on the human or animal body.

## Description

### FIELD OF THE INVENTION

The present invention relates to stable atomic quantum clusters, AQCs, and to their use as chemotherapy agents in human and animal diseases.

### BACKGROUND OF THE INVENTION

There is a family of products for the prevention and treatment of cell proliferative disorders based on the inhibition of topoisomerases. Topoisomerases are cellular enzymes responsible for the winding and unwinding of DNA. If the DNA cannot be unwound the transcription of the DNA message cannot occur and protein will not be synthesized, giving as a result the eventual cell death. Cells that are dividing at rapid rate, such as proliferative tumour cells, are particularly sensitive to topoisomerase inhibitors as their DNA is constantly being unwound in order to be replicated for daughter cells. Thus, DNA, DNA/topoisomerase complex and/or topoisomerases have become popular targets for cell proliferative disorders chemotherapy treatment (Methods in Molecular Biology, 1999, Volume 94). Examples of topoisomerase inhibitors according to their target and mechanism of action are: camptothecine (irinotecan), a topoisomersase I (topo I) inhibitor which binds covalently with hydrogen bonds to the topo I and DNA forming a ternary complex; doxorubicine, a topoisomersase II (topo II) inhibitor which acts interacting with DNA as intercalating agent; and etoposide, topo II inhibitor which binds to the enzyme.

The topoisomerase inhibitors used in clinical have an effective range 5-20 µM. At these concentrations they have significantly toxicity. Therefore, new topoisomerase inhibitors with a greater activity are needed.

Agents that bind to DNA can bind it covalently or non-covalently. The two most likely binding modes for agents wich bind to DNA non-covalently are groove binding and intercalation. "Intercalation" refers to the reversible or irreversible inclusion of, for example, a molecule between two other molecules. Of significance to the present invention, a large class of molecules, mostly polycyclic, aromatic, and planar, are known that intercalate into a double-strand polynucleotide between two adjacent base pairs. This fact results in a substantial change in DNA structure, and causes lengthening, stiffening and unwinding of the helix. Examples of intercalators known in the art include ethidium, proflavin, daunomycin, doxorubicin, and thalidomide. As said, intercalators in general induce local structural changes into the double-stranded polynucleotide which lead to functional changes, often the inhibition of transcription and replication processes due to malfunction of topoisomerase with the modified DNA. DNA intercalation occurs when compounds of an appropriate size and chemical nature fit themselves in between adjacent base pairs of DNA, being the distance between base pairs about 3.4 Angstroms.

On the other hand, the antimicrobial activity of metals and metal ions has been known for a long time and is the basis of the development of many metal coordination therapeutic agents. Besides the use of heavy metals as antibiotics, they are also employed in the design of cytotoxic compounds. For example, the current treatments for cell proliferative diseases such as cancer employ metal coordination complexes, i.e., complexes with ionized metals, where such complexes inhibit DNA replication and cell division but not much has been reported about zero-valent cytotoxic compounds.

US2005/0287225 discloses metal cluster nanocompounds (MₙLₘ) which consist at least of 30 (n=30), preferably 55 (n=55), transition metal atoms (M) (zero-valent), preferably Au, at their metal core, sourrounded or saturated by ligands (L), suitable for the prophylactic and/or therapeutic (curative) treatment of cancerous diseases. The selection for the average size of the metal cores of the metal cluster nanocompounds is such that they are able to attach to the major grooves of the DNA molecules, in particular of B-DNA of the tumor or cancer cells, by forming physical and/or chemical bonds.

Patent application EP 1914196 describes a process for obtaining a family of zero-valent metals denominated AQCs, atomic quantum clusters, and identifies particles with sizes less than 1 nm - 2 nm of various metals. Also described are how to separate, stabilize and use them; the details of the method indicate that the physico-chemical properties of the clusters synthesized by this process are different from nanoparticles (particles larger than 1 nm - 2 nm). The AQCs stop behaving in a "metallic" way and their behaviour becomes molecular in nature. Thus new properties appear in these clusters that are not observed in nanoparticles, microparticles or bulk metal materials. Because the physico-chemical properties of AQCs cannot be simply extrapolated from that of nano/microparticles, then the properties shown by these clusters cannot in principle be predicted from the properties shown by nano/microparticles. The AQCs (in contrast to nanoparticles that require "stabilisation" for charge or steric hindrance by some protective molecule) exhibit extraordinary stability. In the document is also described the use of the mentioned AQCs for *in vitro* assays on cultures of MCF7 lines of breast cancer, finding that its effects were comparable to those obtained with Puromycin, an aminonucleoside antibiotic produced by the bacterium *Streptomyces alboniger.* Puromycin inhibits protein synthesis by disrupting peptide transfer on ribosomes causing premature chain termination during translation.

At the patent application EP2206503 these AQCs were used as antimicrobial agents and biocides.

### BRIEF DESCRIPTION OF THE INVENTION

The inventors of the present invention have surprisingly found that atomic quantum clusters, AQCs, having especial dimensions, present, among others, topoisomerase inhibition abilities which considerably extend their properties and applications in the prevention and/or treatment of cell proliferative disorders, viral infections and autoimmune diseases. These new properties and applications were not evident for the AQCs of the invention before the present discovery of their topoisomerase inhibition abilities.

In one aspect, the invention provides stable atomic quantum clusters, AQCs, pharmaceutically acceptable salts, derivatives and hydrates thereof, characterized in that they have a thickness of less than 0.5 nm for use in the prevention and/or treatment of viral infections, autoimmune diseases and cell proliferative disorders, with the exclusion of breast cancer, on the human or animal body.

In another aspect of the present invention, the prevention and treatment of cell proliferative disorders is performed as a combination therapy of an AQC of the invention with at least one antineoplasic, immunosuppressive or antiviral drug, on the human or animal body.

In a further aspect, the present invention relates to a pharmaceutical composition comprising an AQC of the invention and at least one antineoplasic, immunosuppressive or antiviral drug.

In another aspect, the invention also relates to a method of treating viral infections, autoimmune diseases and cell proliferative disorders, with the exclusion of breast cancer, comprising administering to a patient in the need of such treatment a therapeutically effective amount of an AQC of the invention or a pharmaceutically acceptable salt, derivative or hydrate thereof.

In a final aspect, the invention also relates to a method of treating cell proliferative disorders, viral infections and autoimmune diseases comprising administering to a patient in the need of such treatment a therapeutically effective amount of an AQC of the invention and at least one antineoplasic, immunosuppressive or antiviral drug.

### BRIEF DESCRIPTION OF THE DRAWINGS

Figure 1. Cluster inhibition of topoisomerase I (TOPO I) activity. Lane 1. Ladder. 2. pBR322. 3. pBR322 + TOPO 1. 4. pBR322 + Ag-clusters 300 pM + TOPO 1. 5.pBR322 + Ag-clusters 250 pM + TOPO 1. 6. pBR322 + Ag-clusters 200 pM + TOPO 1. 7. pBR322 + Ag-clusters 150 pM + TOPO 1. 8. pBR322 + Ag-clusters 100 pM + TOPO 1. 9.pBR322 + Ag-clusters 50 pM + TOPO 1. 10. pBR322 + Ag-clusters 25 pM + TOPO I.
Figure 2. Cluster inhibition of topoisomerase II (TOPO II) activity. Lane 1. kDNA + TOPOII. Lane 2. kDNA + Clusters 400 pM + TOPO II. Lane 3. kDNA + Clusters 200 pM + TOPO II. Lane 4. kDNA + Clusters 100 pM + TOPO II. Lane 5. kDNA + Clusters 50 pM + TOPO II. Lane 6. kDNA + Clusters 25 pM + TOPO II. Lane 7. kDNA + Clusters 12,5 pM+ TOPO II. Lane 8. kDNA + Doxorubicin 1µM+ TOPO II.
Figure 3. Effect of clusters on human lung carcinoma cell proliferation (A549). 5000 A549 cells (T0) were incubated in the presence (Ag 0.027 µM) and absence (A549 T48) of Ag clusters for 48 hours.
Figure 4. Cell viability in the presence of Ag AQCs. A549 cells 7 days after treatment with AQCs.
Figure 5. Flow cytometry (a) control; (b) with camptothecin; (c) with AQC; (d) with AQC and Camptothecin.
Figure 6. Dose - response curves
Figure 7. Spectrophotometric titration of CT-DNA/Ag03, I = 0.1 M (NaCl), pH =7.0 (sodium cacodilate), T = 25°C. C⁰ DNA = 5.112×10⁻⁴ M, C⁰_{Ag03} = 4.28 X 10⁻⁸ M. From bottom to top, C _{DNA}/C _{Ag03} = 0-6900.
Figure 8. Deconvolution spectra of CT-DNA/Ag03, *I*= 0.1 M (NaCl), pH =7.0 (sodium cacodilate), T = 25 °C. C⁰ _{DNA} = 5.112×10⁻⁴ M, C⁰_{Ag03} = 4.28 X 10⁻⁸ M, C _{DNA}/C_{Ag03} = 0-6900 (from bottom to top at 300 nm).
Figure 9. Circular dichroism of CT-DNA/Ag03 pH = 7.0, I = 0.1M (NaCl) T = 25 °C, C⁰_{DNA} = 9.76 X 10⁻⁵ M; C_{Ag03} from 0 to 3.71 X 10⁻⁷ M.
Figure 10. Molar ellipticity at 275 nm vs C_{D}

### DETAILED DESCRIPTION OF THE INVENTION

In accordance with the first aspect mentioned above, the present invention refers to a stable atomic quantum cluster (AQC) a pharmaceutically acceptable salt, a derivative or a hydrate thereof, characterized in that said AQC, pharmaceutically acceptable salt, derivative or hydrate thereof has a thickness of less than 0.50 nm, for use as chemotherapy agent in the prevention and treatment of viral infections, autoimmune diseases and cell proliferative disorders, with the exclusion of breast cancer, on the human or animal body.

The term "atomic quantum cluster" or "AQC" means, in accordance with the present invention, a group of two or more transition metal atoms. Transition metal atoms are understood to be zero-valent. The AQC compounds consist thus of transition metal atoms of identical (mononuclear clusters) or different (heteronuclear clusters) transition metals. The atomic quantum cluster according to the invention may also include pharmaceutically acceptable salts, hydrates and derivatives thereof, even if they are not specifically mentioned in the present text. The atomic quantum clusters are also known as "metal quantum cluster".

In the present invention, "stable AQC(s)" is (are) understood as those groupings of metal atoms (AQCs) which conserve the number of atoms and, therefore, their properties, overtime, so that they can be isolated and manipulated like any other chemical compound. The AQCs are conserved for months and even years without the need of an external stabilizer.

Even the AQCs are stable *per se* it is possible that they comprise one or more ligands or ions, thus forming what is called AQCs derivatives or AQCs salts respectively, which may act as a stabilizing or solubilizing agents.

The term "derivative" as used in this document is defined here as meaning an AQC which comprises one ore more ligands to change (for pharmaceutical use) any of its physico-chemical properties, such as solubility or bioavailability.

The ligands are organic compounds including functional groups, preferably two functional groups. In a preferred embodiment, one functional group links the ligand to the cluster, whereas the other helps to solubilize it. Non-limitative examples of the functional groups are amines, acids, esters, hydroxyl, amicoacids, etc.

The term "pharmaceutically acceptable salt" refers to any pharmaceutically acceptable salt which, upon administration to the patient is capable of providing (directly or indirectly) a compound as described herein. Examples of the acid addition salts include mineral acid addition salts such as, for example, hydrochloride, hydrobromide, hydroiodide, sulphate, nitrate, phosphate, and organic acid addition salts such as, for example, acetate, trifluoroacetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium and ammonium salts, and organic alkali salts such as, for example, ethylenediamine, ethanolamine, N,N-dialkylenethanolamine, triethanolamine and basic aminoacids salts. However, it will be appreciated that non-pharmaceutically acceptable salts also fall within the scope of the invention since those may be useful in the preparation of pharmaceutically acceptable salts.

The term "pharmaceutically acceptable" refers to compositions and molecular entities that are physiologically tolerable and do not typically produce an allergic reaction or a similar unfavorable reaction as gastric disorders, dizziness and suchlike, when administered to a human or animal. Preferably, the term "pharmaceutically acceptable" means it is approved by a regulatory agency of a state or federal government or is included in the U.S. Pharmacopoeia or other generally recognized pharmacopoeia for use in animals, and more particularly in humans.

The term "hydrate" according to this invention is to be understood as meaning any form of the AQC according to the invention which has water molecules attached to it via non-covalent bonding.

It is compulsory, in the context of the present invention, that the size of the AQCs derivatives, salts or hydrates, allows the intercalation or the partial intercalation in DNA, which means that the final thickness of the AQC derivatives, salts or hydrates is less of 0.50 nm, preferably less than 0.45 nm and more preferably less than 0.34 nm. The size of the AQCs of the invention can be measured by HR-STEM (high-resolution scanning transmisión electron microscopy), by AFM (atomic force microscopy) or by DLS (dynamic light scattering).

In a preferred embodiment, the present AQCs are composed of less than 25 metal atoms (Mₙ, n<25), preferably 12 metal atoms (Mₙ, n<12) and, more preferably, the present AQCs are formed by between 2 and 5 metal atoms (Mₙ, 2<n<5) and even more preferably they are formed by 2 or 3 metal atoms. In another embodiment the AQCs of the invention are composed of less than 12 metal atoms and have planar or almost planar geometry.

In a further preferred embodiment, the metals (M) wherefrom those AQCs are formed are selected from platinum (Pt), gold (Au), rhodium (Rh), iridium (Ir), palladium (Pd), ruthenium (Ru), osmium (Os), silver (Ag), copper (Cu), iron (Fe), cobalt (Co), nickel (Ni), titanium (Ti), vanadium (V), chrome (Cr) or their bi and multimetal combinations. Preferably the metals of the AQCs are selected from Au, Ag, Cu, Pd and Pt or their bimetal combinations. And more preferably the metals of the AQCs are selected from Au and Ag or their bimetal combinations.

AQCs of the invention have a thickness of less than 0.50 nm, preferably less than 0.45 nm and more preferably less than 0.34 nm. The thickness of the AQCs in the range mentioned is necessary in order to make possible the intercalation or partial intercalation of the AQCs in the DNA. The distance between base pairs of DNA is 0.34 nm and the AQCs are designed to be situated between them to be useful as intercalators. For the same reason, the size of the AQCs of the invention has a limitation of the thickness but there is no limitation of the breadth. This definition makes that the metal atoms are preferably disposed on the range of the same plane or almost the same plane. Thus, in a preferred embodiment the AQCs of the invention are planar or almost planar. In another embodiment, the geometry of the AQCs is different than planar and the thickness less of 0.50 nm, preferably less than 0.45 nm, and more preferably less than 0.34 nm.

By "planar" or "almost planar" it is understood in the context of the present invention to compositions and molecular entities, which are of sufficient planarity to be intercalated, at least partially in the DNA.

The preparation of the AQCs, salts, hydrates and derivatives thereof, as well as AQCs including ligands according to the present invention can be performed, as described in EP 2206503, for example for Ag AQCs, in an electrochemical cell using galvanostatic potentiometry, applying a constant current density of 0.2 mA/cm² for different times (t) under the following experimental conditions: Working electrode: Pt (6 cm2). Contraelectrode: Ag. Reference electrode: Ag/AgCl. Electrolyte solution: 200 µM tetrabutylammonium bromide in water. Temperature: 25 °C. The final samples were diluted in water to obtain a final concentration of 100 nM clusters in atoms of Ag.

The present AQCs inhibit the topoisomerases I and II. This fact has been proved in both activity assays at example 2 wherein the activity of topoisomerase I and/or II was inhibited in the presence of AQCs of the invention. Supercoiled DNA pBR322 is transformed to relaxed DNA by Topoisomerase I, but in the presence of increasing concentrations of AQCs there is a reduction of the formation of the relaxed DNA, as it is shown by the agarose gel, and the supercoiled band of pBR322 appears as if no, or few, Topoisomesae I was present, proving the inhibition power of the AQCs over the Topoisomerase I. Analogously, kDNA is transformed by topoisomerase II to NOC (*Nicked open circular*) DNA and CCC (*covalently closed circular*) DNA, but in the presence, as well, of increasing concentrations of AQCs, these substrates are decreasingly formed due to the inhibition of the Topoisomerase II by the AQCs. These results represent that the DNA cannot be unwound by the topoisomerases in the presence of a sufficient amount of AQCs. The term "sufficient" in this context means the minimum concentration of AQC at which all or part of DNA remains with its original structure in the presence of topoisomerase I or II and AQC.

The changes provoqued by intercalation in DNA are evaluated by dichroism and electronic absorption spectroscopy methods in the examples, from which is possible to further distinguish intercalation from groove binding. As shown in the examples of the present patent application, the AQCs of the invention inhibit the topoisomerases I and II by DNA intercalation, more particularly by partial intercalation. This fact provoques, as have been comented above, that DNA cannot be unwound and DNA replication cannot be performed, giving as a result the eventual cell death or apoptosis.

Furthermore, the Ames test, example 5, well known in the art, has been performed showing that AQCs are not mutagenic, i.e. the AQCs are not capable to change the content of DNA.

As "chemotherapy agents" as described herein are drugs for inhibiting or suppressing cellular growth, multiplication and proliferation, as well as inhibiting viral replication.

A "cell proliferative disorder" as described herein may be a neoplasm. The term "neoplasm" refers to a new, abnormal growth of cells or a growth of abnormal cells without physiological control. A neoplasm creates an unstructured mass (a tumor). In preferred embodiments, the AQCs of the invention are used to treat cell proliferative disorders including but not limited to primary tumors, metastases and precancerous conditions (pre-cancer stages). Examples of cell proliferative disorders are, but are not limited to, spleen, colorectal and/or colon cancer, colon carcinomas, ovarian carcinomas, ovarian cancer, breast cancer, carcinomas of the uterus, lung cancer, stomach cancer, esophageal cancer, liver cancer, carcinomas of the pancreas, kidney cancer, bladder cancer, prostate cancer, testicular cancer, bone cancer, skin cancer, sarcoma, Kaposi sarcomas, brain tumours, myosarcomas, neuroblastomas, lymphomas and leukemias; melanoma, glioma, medulloblastoma, head and neck carcinoma.

As autoimmune disease is understood a disease caused by the action of the immune system against the body's own substances, tissues or organs. It also includes the rejection of a tissue or an organ transplanted from a donor. When an autoimmune disease occurs, there is an overactive response of immune system cells. The AQCs of the invention are meant to decrease the proliferative growth of the immune system cells by inhibiting the topoisomerases I and/or II as previously described.

In a preferred embodiment, the AQCs of the invention are used to treat autoimmune diseases including but not limited to multiple sclerosis, dermatomyositis, polymyositis, lupus, rheumatoid arthritis and the suppression of transplant rejections.

As "viral infection" is understood in this invention the diseases caused by the entry of a virus in the human or animal cells, from now "host cells" wherein the virus is DNA-virus or RNA-virus. Once a host cell is infected, the virus can remain latent or it can begin making copies of itself, then, the replication occurs quickly. The AQCs of the invention are meant to inhibit the viral replication of virus infected cells by intercalating the viral DNA and thus inhibiting the topoisomerases I and/or II, as previously described, and/or by inhibiting any other protein which may interact with DNA for its replication. Furthermore, without the need to be bound by a theory, it is belived that the AQCs of the invention inhibit the integrase family of viral enzymes due to the conservation of structure and mechanism between Eukaryotic Topoisomerase I and the integrase family.

Thus, the AQCs of the invention are used to treat viral infenctions diseases including infections caused by DNA viruses, RNA viruses including retrovirus. The skilled person knows examples of DNA viruses and RNA viruses.

In a preferred embodiment, the AQCs of the invention are used to treat viral infections including but not limited to Acquired Immunodeficiency Syndrome, Bronchiolitis, Chicken Pox, Common Cold, Conjunctivitis, Coxsackie Virus, Creutzfeldt-Jakob Disease, Croup, Cytomegalovirus Infections , Dengue, Ebola, Encephalitis, Epstein-Barr Virus, Foot (Hoof) and Mouth Disease, Hand, Foot and Mouth Disease, Hantavirus, Hemorrhagic Fevers, Hepatitis, Herpes, Human Papillomavirus, Influenza (Flu), La Crosse Encephalitis, Lassa Fever, Marburg Hemorrhagic Fever, Measles (Rubeola), Meningitis, Monkeypox, Mononucleosis Infectious, Mumps, Norwalk Virus Infection, Orchitis, Poliomelitis, Progressive Multifocal Leukoencephalopathy, Rabies, Respiratory Syncytial Virus, Rotavirus, Rubella (German Measles), Severe Acute Respiratory Syndrome (SARS), Shingles (Herpes Zoster), Smallpox, Viral Hemorrhagic Fevers, Viral Pneumonia, Encephalitis and Yellow Fever.

The pharmaceutical composition of the invention, obtained by mixing the AQC(s) with the suitable pharmaceutically aceptable carrier(s) may be administered in a plurality of pharmaceutical forms of administration, e.g. solid (tablets, pills, capsules, granules etc.) or liquid (solutions, suspensions or emulsions).

The term "carrier" refers to a diluent, adjuvant, excipient, or vehicle with which the active ingredient is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E.W. Martin.

Administration may be carried out, for example, orally, lingually, sublingually, buccally, rectally or parenterally (i.e. by circumventing the intestinal tract, i.e. intravenously, intraarterially, intracardially, intracutaneously, subcutaneously, transdermally, intraperitoneally or intramuscularly), with oral and intravenous administration being particularly suitable; very particular preference is given to oral administration.

A topical application is also possible (e.g. for the treatment of melanomas). A particular form of topical application consists of introducing AQC of the invention and at least an antineoplasic or an antiviral drug into a carrier system, in particular a drug delivery system, and implanting said carrier system into the neoplastic or cancerous tissue or at least close to or in the environment of said neoplastic or cancerous tissue or viral infected tissue, where said carrier system then releases said AQC of the invention and the antineoplasic drug or an antiviral drug specifically at the site of said neoplastic or canceraus tissue or viral infected tissue. In this way it is possible to avoid side effects, as may occur in the case of systemic administration, i.e. to reduce the overall strain on the body markedly.

For a better formulation of the present invention, dispersants can be also added, such as surfactants and/or polymers.

Non-limitative examples of surfactants include:
a) non-ionic surfactants: saturated linear long chain fatty alcohols, as lauryl alcohol, cetyl alcohol; branched chain fatty alcohols; sterol alcohols such as cholesterol; partial esters of fatty acids of multivalent alcohols as ethylene glycol monostearate, glicerin monoestearate, glycerin monooleate; partial esters of fatty acids of sorbitan such as sorbitan monolaurate, sorbitan monopalmitate, sorbitan monooleate; partial esters of fatty acids of polioxietilensorbitan, such as polyoxyethylene (20) sorbitan monolaurate, polyoxyethylene sorbitan monooleate, sorbitol eters with polyoxyethylene such as polyoxyethylene sorbitol oleate-laurate, polyoxyethylene sorbitol hexaoleate; eters of fatty acids with polyoxyethylene such as polyoxyethylene oleyl ether, polyoxyethylene (10) cetil eter; esters of fatty acids with sucrose such as sucrose monolaurate; polyglycerol esters of fatty acids; eters CᵢEⱼ type, (i= 4-16, j = 1-4), as C₄E₂ = diethylene glycol monobutyl eter, C₆E₃= triethyleneglycol monohexil eter, and others, such as polyoxyethylene nonylphenyl ether; pentaethylene glycol mono nonyl phenyl ether, N-tetraethylenepentamine polybutenyl succinimide.
b) anionic surfactants: alkaline and metalic soaps such as sodium oleate, calcium palmitate, aluminium stearate; amino soaps as triethanolamine stearate and sulfonated combinations as sodium lauryl sulfate, sodium bis(2-ethylhexyl) sulfosuccinate, sodium cetyl sulfonate ; bile acid salts, as sodium glycocholate; gum arabic.
c) cationic surfactants, such as cetylpyridinium chloride; cetil tributylammonium bromide.
d) amphoteric surfactants, such as proteins or lecithin.

Non-limitative examples of polymers include polyvinylpyrrolidone (PVP), polylactic acid (PAA), polyethylene glycol (PEG) and block copolymers, such as those formed by the above polymers and polymethacrylic acid, polyurethane, polyaniline and/or polyester.

Other dispersants not included in the above also include dextrans, dendrimers, carbohydrates, etc.

Generally a therapeutically effective administered amount of the AQC compounds of the invention will depend on the relative efficacy of the compound chosen, the severity of the disorder being treated and the weight of the sufferer. However, AQC active compounds will typically be administered once or more times a day for example 1, 2, 3 or 4 times daily, with typical total daily doses in the range of from 0.0001 to about 500 mg/kg/day, in particular from about 0.0001 to about 100 mg/kg/day, preferably 0.01 to 50 mg/kg/day.

The term "therapeutically effective amount" refers to a quantity that produces a result that in and of itself helps to heal or cure.

In another aspect, the present invention relates to a stable atomic quantum cluster (AQC), a pharmaceutically acceptable salt, a derivative or a hydrate thereof, characterized in that said AQC, pharmaceutically acceptable salt, derivative or hydrate thereof has a thickness of less than 0.50 nm, preferably less than 0.45 nm, and more preferably less than 0.34 nm, for use as chemotherapy agent in the prevention and treatment of cell proliferative disorders in combination therapy with at least one antineoplasic, immunosuppressive or antiviral drug, on the human or animal body.

Examples of antineoplasic drugs are, but are not limited to, nitrogen mustards: cyclophosphamide, mustine, uramustine, melphalan, chlorambucil, ifosfamide; nitrosoureas: carmustine, lomustine, streptozocin; alkyl sulfonates: busulfan; daunorubicin; anthracyclines: idarubicin, danurubicin, epirubicin; platinums: cisplatin, carboplatin, nedaplatin, oxaliplatin, satraplatin, tetraplatin tetranitrate; kinase inhibitors: imatinib, erlotinib, gefitinib, sorafenib tosylate, sunitinib; monoclonal antibodies: trastuzumab, cetuximab, bortezomib, bezacizumab; cytosine arabinoside, fludarabine, decitabine, clofarabine, nelarabine; antimetabolites: capecitabine, gemcitabine, pemetrexed; plant alkaloids: taxanes, docetaxel, paclitaxel; vinca alkaloids: vinblastine, vincristine and vinorelbine; and campthotecins.

Examples of immunosuppressive drugs are, but are not limited to, cortisol, dexamethasone, cyclophosphamide, methotrexate, azathioprine dactinomycin, antibodies, ciclosporin, tacrolimus, sirolimus and opioids.

Examples of antiviral drugs are, but are not limited to, acyclovir, adefovir, amantadine, cidofovir, darunavir, efavirenz, emtricitabine, enfurtivide, lamiducine, maraviroc, methisazone, nevirapine, ostealmivir, pleconaril, ralegravir, ritonavir, tenofovir, zanavir and zidovudine.

Said combination therapy may be performed in a simultaneous, separate or sequencial administration.

By "simultaneous", within the meaning of the present invention is meant in the present application an administration of at least 2 active ingredients, the AQC of the invention and at least one antineoplasic, immunosuppressive or antiviral drug, by the same route and at the same time or at substantially the same time.

By "separate" use, within the meaning of the present invention is meant in particular an administration of at least 2 active ingredients, the AQC of the invention and at least one antineoplasic, immunosuppressive or antiviral drug, at the same time or at substantially the same time by different routes.

By "sequential" use is meant an administration of at least 2 active ingredients, the AQC of the invention and at least one antineoplasic, immunosuppressive or antiviral drug, at different times, the administration route being identical or different. More particularly by an administration method is meant according to which the whole administration of one of the active ingredients is carried out before administration of the other or others commences. It is thus possible to administer one of the active ingredients over several months before administering the other active ingredient or ingredients. There is no simultaneous treatment in this case. An alternate administration of each active ingredient over several weeks can also be envisaged.

In another aspect the invention relates to a pharmaceutical composition comprising an AQC of the invention and at least one antineoplasic, immunosuppressive or antiviral drug. In a particular embodiment, said compounds may be present in the form of a mixture or spatially separated from another, either forming part of the same dosage form or as a kit of parts.

By "mixture" within the meaning of the present invention is meant that the components of the combination, the AQC of the invention and at least one antineoplasic, immunosuppressive or antiviral drug, are mixed together and provided in a common presentation form, e.g., tablet.

By "spatially separated" within the meaning of the present invention is meant that the components of the combination, the AQC of the invention and at least one antineoplasic, immunosuppressive or antiviral drug, are separated by an arbitrarily small distance, as in a single dosage form, e.g., layered tablets or coated-core tablets; or as in a kit of parts.

The pharmaceutical composition comprising an AQC of the invention and at least one antineoplasic, immunosuppressive or antiviral drug used according to the invention may be administered systematically or else topically, in particular locally, depending on the type of the disorders to be treated.

Any customary forms of administration are suitable for administering the pharmaceutical composition comprising an AQC of the invention and at least one antineoplasic, immunosuppressive or antiviral drug used according to the invention. Administration may be carried out, for example, orally, lingually, sublingually, buccally, rectally or parenterally (i.e. by circumventing the intestinal tract, i.e. intravenously, intraarterially, intracardially, intracutaneously, subcutaneously, transdermally, intraperitoneally or intramuscularly), with oral and intravenous administration being particularly suitable; very particular preference is given to oral administration. A topical application is also possible (e.g. for the treatment of melanomas).

A particular form of topical application consists of introducing AQC of the invention and at least one antineoplasic, or antiviral drug into a carrier system, in particular a drug delivery system, and implanting said carrier system into the neoplastic or cancerous tissue or infected tissue or at least close to or in the environment of said neoplastic or cancerous tissue or infected tissue, where said carrier system then releases said AQC of the invention and the at least one antineoplasic or an antiviral drug specifically at the site of said neoplastic or canceraus tissue or infected tissue. In this way it is possible to avoid side effects, as may occur in the case of systemic administration, i.e. to reduce the overall strain on the body markedly. Examples of implantable carrier or drug delivery systems suitable according to the invention are described in the international laid-open publication WO 00/25841 A1.

For application according to the invention, the AQC of the invention and at least one antineoplasic, an immunosuppressive or an antiviral drug are transferred into the usual formulations such as, for example, tablets, sugar-coated tablets, pills, granules, aerosols, syrups, emulsions, suspensions, solutions, ointments, creams and gels of any kind, in particular by using inert, essentially nontoxic, pharmaceutically suitable carriers or solvents. To this end, an AQC and at least one antineoplasic, immunosuppressive or antiviral drug used according to the invention may be present in each case at a therapeutically active concentration, in particular at concentrations of from about 0.0001 to about 99% by weight, preferably from about 0.01 to about 95% by weight, of the total mixture, i.e. in amounts sufficient to achieve the indicated or desired dosage range. Nevertheless, it may be necessary, where appropriate, to deviate from the above mentioned amounts, namely depending on the body weight or on the type of route of administration, on the individual reaction to the medicament, on the type of formulation and on the time or interval of administration. Thus it may be sufficient, in some cases, to manage with less than the aforementioned minimal amount, while in other cases the upper limit mentioned has to be exceeded. In the case of administering relatively large amounts, it may be recommended to distribute said amounts in the form of several single doses over a defined period of time, for example during the day.

In another embodiment of the present invention the inventive combination may contain AQC and at least one antineoplasic, immunosuppressive or antiviral drug essentially in an equieffective ratio.

In yet a further embodiment of the inventive combination AQC and at least one antineoplasic, immunosuppressive or antiviral drug are present in such a weight ratio that the resulting composition will exert a synergistic effect upon administration to a patient. Suitable weight ratios can be determined by methods well known to those skilled in the art.

Both components, AQC and at least one antineoplasic, immunosuppressive or antiviral drug, may also be present in the inventive combination in ratios deviating from the equieffective ratio. For, example, each of the components could be present in a range from 1/50 of the equieffective amount to 50 times the equieffective amount, from 1/20 of the equieffective amount to 20 times the equieffective amount, from 1/10 of the equieffective amount to 10 times the equieffective amount, from 1/5 of the equieffective amount to 5 times the equieffective amount, from 1/4 of the equieffective amount to 4 times the equieffective amount, from 1/3 of the equieffective amount to 3 times the equieffective amount, or from 1/2 of the equieffective amount to 2 times the equieffective amount.

The formulations are prepared, for example, by diluting, the AQC of the invention and at least one antineoplasic, immunosuppressive or antiviral drug with solvents (e.g. oils such as castor oil) and/or carriers, where appropriate by using emulsifiers and/or dispersants, it being possible, for example in the case of utilizing water as a diluent, to use, where appropriate, organic solvents as auxiliary solvents.

Depending on the type of administration, it has proved advantageous to administer the active compounds or active compound combinations used according to the invention in amounts of from about 0.0001 to about 500 mg/kg of body weight, in particular from about 0.0001 to about 100 mg/kg, preferably 0.01 to 50 mg/kg, in order to achieve more effective results. Nevertheless, it may be necessary, where appropriate, to deviate from the abovementioned amounts, namely depending on the body weight or on the type of route of administration, on the individual reaction to the medicament, on the type of formulation and on the time or interval of administration. Thus it may be sufficient, in some cases, to manage with less than the aforementioned minimal amount, while in other cases the upper limit mentioned has to be exceeded. In the case of administering relatively large amounts, it may be recommended to distribute said amounts over a defined period of time, for example during the day, that is, for example, in the form of several single doses or of continuous administration (e.g. continuous infusion). The application in a chronic therapy (e.g. in tablet form) is likewise possible.

In another aspect the invention is related to a method of treating cell proliferative disorders comprising administering to a patient in need of such treatment a therapeutically effective amount of AQC, pharmaceutically acceptable salts, derivatives and hydrates thereof, and a therapeutically effective amount of at least one chemotherapeuthic drug. Said administration may be performed in a simultaneous, separate or sequencial administration.

In another aspect the invention is related to a method of treating autoimmune diseases comprising administering to a patient in need of such treatment a therapeutically effective amount of AQC, pharmaceutically acceptable salts, derivatives and hydrates thereof, and a therapeutically effective amount of at least one immunosupresive drug. Said administration may be performed in a simultaneous, separate or sequencial administration.

In another aspect the invention is related to a method of treating viral infections comprising administering to a patient in need of such treatment a therapeutically effective amount of AQC, pharmaceutically acceptable salts, derivatives and hydrates thereof, and a therapeutically effective amount of at least one antiviral drug. Said administration may be performed in a simultaneous, separate or sequencial administration.

The invention will be illustrated below by tests performed by the inventors.

### EXAMPLES

EXAMPLE 1. Synthesis of Ag AQCs.

The Ag clusters (Ag AQCs) used throughout the experiments were synthesized, as described above, in an electrochemical cell using galvanostatic potentiometry, applying a constant current density of 0.2 mA/cm² for 80 minutes at 25 °C under the following experimental conditions: Working electrode: Pt (6 cm2). Contraelectrode: Ag. Reference electrode: Ag/AgCl. Electrolyte solution: 200 µM tetrabutylammonium bromide in water. The final sample was diluted in water to obtain a final concentration of 100 nM clusters in atoms of Ag. The size of the obtained AQCs is 0.3 nm having a planar symmetry and containing 3 atoms of Ag (Ag03). The stability of the Ag03 AQC is of at least one year in the absence of stabilizing ligands.

### EXAMPLE 2. Topoisomerase inhibition

Human topoisomerase I and II activity assay: The activity of the enzymes was assessed using a commercial kit (Inspiralis, UK) as described by the manufacturer.

DNA was preincubated with clusters at room temperature and then different human recombinant enzymes were added. The reaction was then followed during 30 minutes at 37°C. Reaction samples were run in agarose gels and topoisomerase inhibition was determined visually.

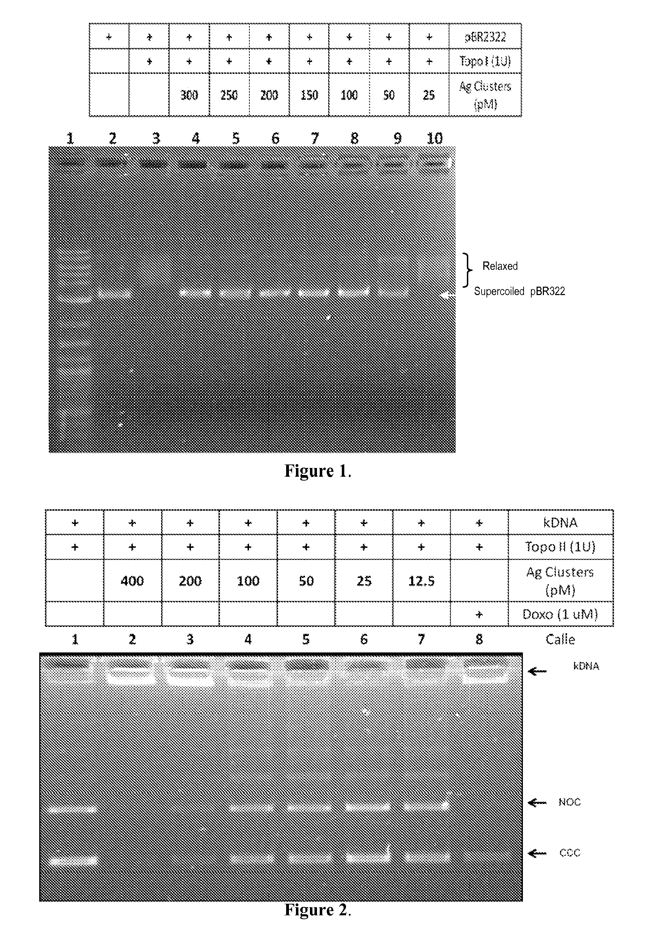

For the assay of the activity of human topoisomerase I the Human Topoisomerase I Relaxation Kit was used. Increasing concentrations of clusters from 25 to 300 pM (lanes 10 to 4) show a dose dependent inhibition of topoisomerase I activity as seen by the reduction of the relaxed band and the appearance of a supercoiled pBR322. (Fig. 1) The inhibition of the decatenation activity of human topoisomerase II by the AQCs was determined using the human topoisomerase II decatenation kit. kDNA is catenated DNA. Increasing concentrations of clusters from 12.5 to 400 pM (lanes 7 to 2) show a dose dependent inhibition of TOPO isomerase II activity as seen by the remaining kDNA and the absence of the NOC and CCC bands (Fig 2). Doxorubicin, a well-known topoisomerase inhibitor was used as control (lane 8).

### EXAMPLE 3. Cell proliferation assay

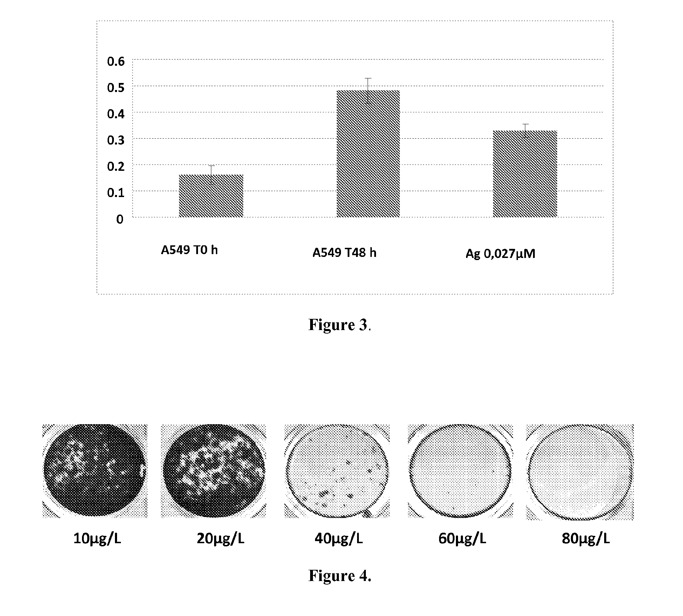

The MTT assay was used to estimate the effect of clusters on cell proliferation. The human lung cell carcinoma line A549 was used to test the effects of clusters on tumor cell proliferation. Cells were incubated in the presence of clusters at various concentrations for 48 hours. After that, the number of remaining cells was assessed by means of the MTT assay. In figure 3 it is shown that the presence of clusters at concentrations about 30 nM inhibits the cell proliferation by 50%.

EXAMPLE 4. Effect of silver clusters on cell viability.

The clusters produce a cytotoxic dose-dependent effect. A549 cells were seeded and 24 hours later were treated by adding different concentrations of AQCs in the culture medium for 4 hours. Figure 4 shows a representative example of the impact of clusters on cell proliferation 7 days after treatment. The cell clones were stained with crystal violet (black dots in the photo).

### EXAMPLE 5. Mutagenic activity

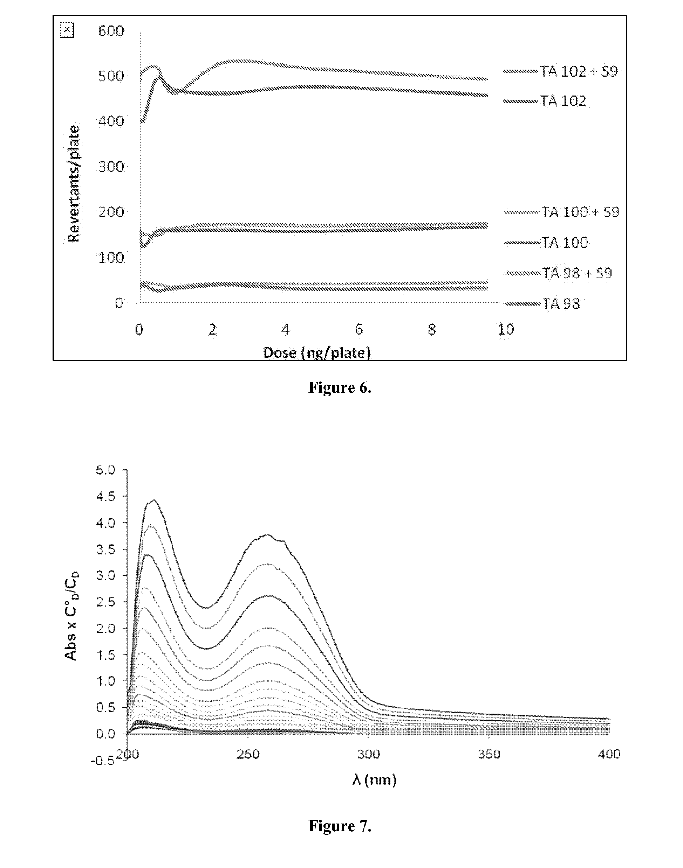

Mutagenic activity was assayed in the Ames Test as has been described elsewhere (Maron, D.M., Ames, B.N., Revised methods for the Salmonella mutagenicity test, Mutation Research, 1983, 113, 173-215), using strains TA 98, TA 100 and TA 102, which were kindly provided by Prof. B. Ames. The test compounds were dissolved in water. Dose-response curves (Figure 6) were generated for each compound using the standard incorporation plate assay, in the presence and in the absence of S9 mix (microsomal fraction of rat liver that contains enzymes related to the metabolic fate of genotoxicants).

The results shown in Table 1 are means of at least two independent experiments. It could be observed a negative result for mutagenic activity on all the strains tested in this assay at the concentration range from 0 to 9.48 ng/plate, both in the presence and in the absence of S9 mix. No cytotoxicity for the doses tested is found in this assay. So it could be concluded that Ago2 has not genotoxic activity on TA 98, TA 100 and TA 102 strains, neither in the presence nor in the absence of S9 mix.

**Table 1: Ames Test on TA 98, TA 100 and TA 102 with and without S9 mix**

| **ng Ag02/plate** | **TA 98** | **TA 98 + S9** | **TA 100** | **TA 100 + S9** | **TA 102** | **TA 102 + S9** |
|---|---|---|---|---|---|---|
| 0.00 | 33 ± 1 | 40 ± 8 | 165 ± 29 | 155 ± 10 | 405 ± 45 | 489 |
| 0.09 | 39 ± 8 | 46 ± 6 | 125 ± 16 | 153 ± 28 | 403 ± 35 | 512 |
| 0.47 | 28 ± 5 | 41 ± 5 | 160 ± 5 | 150 ± 32 | 497 ± 7 | 519 |
| 0.95 | 33 ± 6 | 37 ± 8 | 160 ± 12 | 166 ± 33 | 471 ± 11 | 464 |
| 2.37 | 40 ± 8 | 44 ± 6 | 161 ± 3 | 174 ± 7 | 462 ± 7 | 532 |
| 4.74 | 31 ± 3 | 41 ± 8 | 158 ± 23 | 171 ± 18 | 478 ± 19 | 517 |
| 9.48 | 33 ± 1 | 46 ± 9 | 168 ± 1 | 176 ± 22 | 459 ± 83 | 494 |

### EXAMPLE 6. UV - VIS and CD spectrometic titration of the CT_ DNA(Calf Thymus-DNA) - Ag02 system

Calf Thymus DNA (CT-DNA) was purchased from Sigma-Aldrich as the lyophilized sodium salts and used without further purification. CT-DNA was disolved in water and sonicated.

DNA sonication was carried out using a MSE-Sonyprep sonicator, by applying to suitable CT-DNA samples (10 mL of CT-DNA ca. 2 × 10⁻³ M_{BP}) seven repeated cycles of 10 s sonication and 20 s pause, at an amplitude of 14 µm. The sonicator tip was introduced directly into the solution, this being kept in an ice bath to minimize thermal effects due to sonication. Agarose gel electrophoresis test indicated that the polymer length was reduced to ca. 1000 base-pairs. The polynucleotide concentration is expressed in molarity of base pairs, M_{BP}, and is indicated as C_{P}.

Spectrophotometric titration weas performed on a Hewlett-Packard 8453A (Agilent Technologies, Palo Alto, California) spectrophotometer outfitted with diode array detection and computer-assisted temperature control systems.

Circular dichroism spectra, CD, were recorded on a MOS-450 Bio-Logic spectrometer (Claix, France). The measurements were performed in 1.0 cm path length cells at 25 °C. Micro amounts of dye (Ag02) were added to a CT-DNA solution.

### EXAMPLE 6A) UV-Vis spectral measurements

Electronic absorption spectroscopy is universally employed to determine the binding constant associated to the interaction of ligands with DNA. The spectrum of the Ag02 was monitored while small amounts of CT-DNA were added. The Figure 7 shows the spectra corrected by dilution factor.

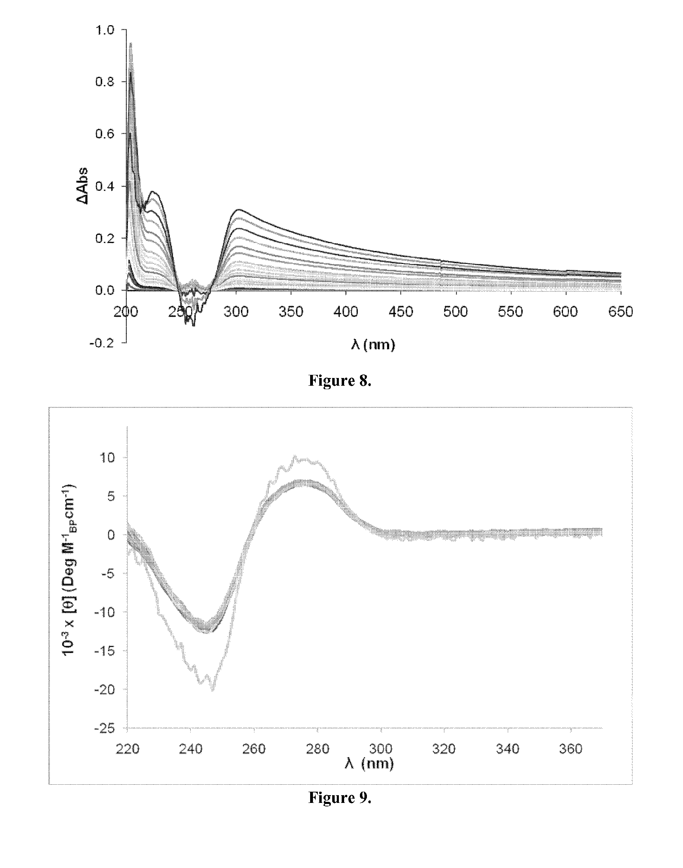

The binding is better understood by observing the corresponding deconvolution (figure 8), where the single contribution of the new formed species (CT-DNA/ Ag02 complex) is calculated by substracting the absorbance of both the ligand and the CT-DNA.

### EXAMPLE 6B. Circular Dicroism (CD)

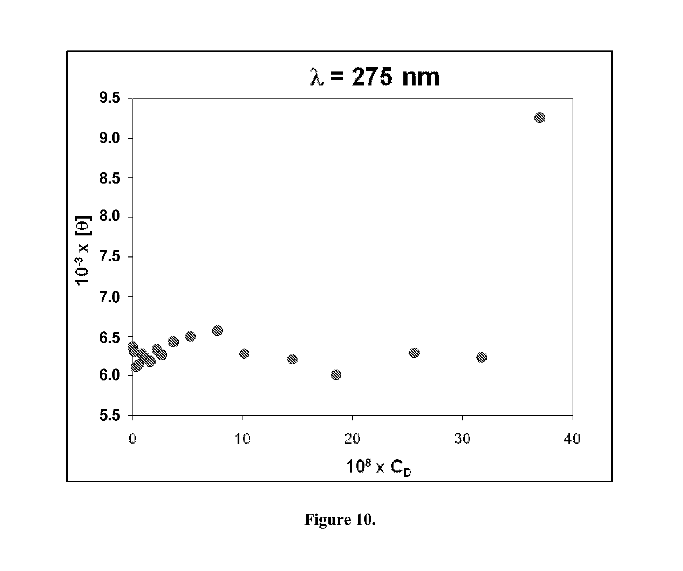

The circular dichroism technique has been applied extensively to investigate the mode of the binding of small ligands (such as dyes and antibiotics) to nucleic acids. Intercalation results in a substantial change in DNA structure (Suh, D.; Chaires, J. Criteria for the mode of binding of DNA binding agents. Bioorg. Med. Chem. 1995, 3 (6), 723-728 and references therein) whereas only subtle changes in structure are observed in groove binding and external binding. The Ag02 ligands studied are achiral clusters. Their interaction with CT-DNA at different C_{D}/C_{P} input ratio is reported in Figure 9. The variation observed at 275 nm shows that the cluster changes the unwinding of the DNA, what is may be due to intercalation processes of the cluster between the bases pair of the DNA. The representation at 275 nm shows that the molar ellipcitity, θ, is affected even with very low concentrations of the cluster (Figure 10).

These results show some hints about the possible mechanism related with the anticancer properties of AQCs. They show that AQCs interact with DNA by partial intercalation.

### EXAMPLE 7.

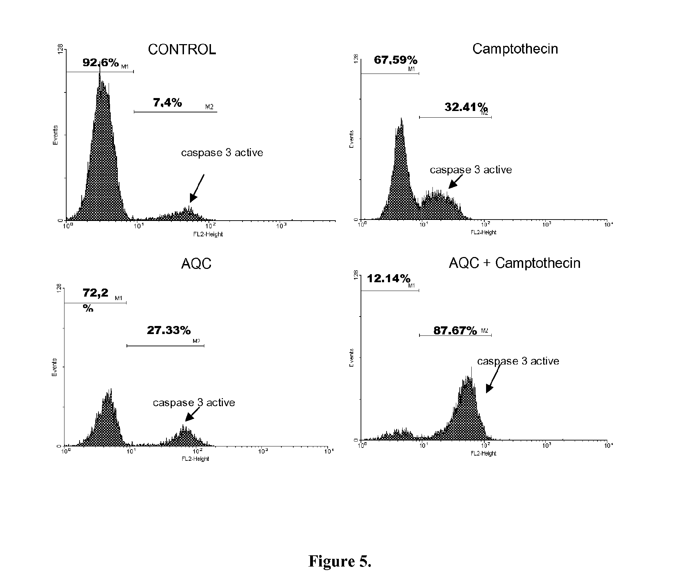

HL-60 cells were incubated with the various treatments. The number of cells that showed the activated form of caspase 3 was assessed by flow cytometry using the "PE Active Caspase-3 Apoptosis kit" (BD. Catalog number 550914). As seen in figure 5, 7.4 % of the non treated cells (control) were positive for activated caspase 3. The percentage of positive cells was increased after camptothecin treatment (30µM) to 32.4 % or clusters treatment (AQC) (200µg/L) to 27.3 % indicating that both treatments were inducing the apoptosis of tumor cells. (It is well known that caspase 3 is the main effector of apoptosis mediated by caspase activation). Interestingly, when both treatments were given together (AQC (200 µg/L) + Camptothecin (30 µM)) the percentange of positive cell was 86,6% indicating that the adittion of clusters and campothecin had a sinergystic effect on caspase 3 activation, and then, on apoptosis.

## Claims

1. A stable atomic quantum cluster (AQC), a pharmaceutically acceptable salt, a derivative or a hydrate thereof, **characterized in that** said AQC, pharmaceutically acceptable salt, derivative or hydrate thereof has a thickness of less than 0.50 nm, for use as chemotherapy agent in the prevention and/or treatment of viral infections, autoimmune diseases and cell proliferative disorders, with the exclusion of breast cancer, on the human or animal body.

2. AQC according to claim 1, wherein its thickness is lower than 0.45 nm, preferably lower than 0.34 nm.

3. AQC according to any of claims 1 to 3 wherein the AQC has planar or almost planar geometry.

4. AQC according to any precedent claim, having less than 25 metal atoms, preferably 12 metal atoms.

5. AQC according to any precedent claim, wherein the metal atoms are selected from platinum (Pt), gold (Au), rhodium (Rh), iridium (Ir), palladium (Pd), ruthenium (Ru), osmium (Os), silver (Ag), copper (Cu), iron (Fe), cobalt (Co), nickel (Ni), titanium (Ti), vanadium (V), chrome (Cr) and mixtures thereof.

6. AQC according to any precedent claim further comprising one or more ligands, wherein the final thickness is less of 0.50 nm.

7. AQC according to claim 6, wherein the ligands are organic compounds including one or two functional gropus.

8. AQC according to any precedent claim, wherein the AQC inhibits the topoisomerases I and II.

9. AQC according to claims 1 to 8 wherein cell proliferative disorders are selected from primary tumors, metastases and precancerous conditions (pre-cancer stages).

10. AQC according to claims 1 to 8 wherein autoimmune diseases are selected from multiple sclerosis, dermatomyositis, polymyositis, lupus, rheumatoid arthritis and the suppression of transplant rejections.

11. AQC according to claims 1 to 8 wherein viral infections are selected from herpes, Chicken Pox, Influenza, Acquired Immunodeficiency Syndrome, Hepatitis, Dengue Fever, Epstein-Barr Virus infection, Hantavirus infection, Severe Acute Respiratory Syndrome, Smallpox and Yellow Fever.

12. AQC, a pharmaceutically acceptable salt, a derivative or a hydrate thereof, as defined in claims 1 to 8, for use as chemotherapy agent in the prevention and/or treatment of cell proliferative disorders in combination therapy with at least one antineoplasic drug, on the human or animal body.

13. AQC, a pharmaceutically acceptable salt, a derivative or a hydrate thereof, as defined in claims 1 to 8, for use as chemotherapy agent in the prevention and/or treatment of viral infections in combination therapy with at least one antiviral drug, on the human or animal body.

14. AQC, a pharmaceutically acceptable salt, a derivative or a hydrate thereof, as defined in claims 1 to 8, for use as chemotherapy agent in the prevention and/or treatment of autoimmune diseases in combination therapy with at least one immunosupresive drug, on the human or animal body.

15. A pharmaceutical composition comprising an AQC, a pharmaceutically acceptable salt, derivative or hydrate thereof as defined in claims 1 to 8 and at least one antineoplasic, immunosuppressive or antiviral drug.
